# EUROPÄISCHE PATENTSCHRIFT

(11) **EP 1 381 863 B1**
(45) Veröffentlichungstag und Bekanntmachung des Hinweises auf die Patenterteilung: **15.11.2006**
(21) Anmeldenummer: 02732658.6
(22) Anmeldetag: 24.04.2002
(51) Int. Cl.: G01N 33/543

(54) **SYSTEM FÜR DIE MEMBRANPERMEATIONSMESSUNG**
SYSTEM FOR MEASURING MEMBRANE PERMEATION
SYSTEME POUR MESURER LA PERMEATION MEMBRANAIRE

(30) Priorität: 27.04.2001 DE 10121903
(43) Veröffentlichungstag der Anmeldung: 21.01.2004
(73) Patentinhaber: Nimbus Biotechnologie Gmbh, 04317 Leipzig (DE)
(72) Erfinder: SCHMITT, Johannes, 04157 Leipzig (DE); NÖLLER, Joachim, 04105 Leipzig (DE)
(74) Vertreter: Patentanwälte Ruff, Wilhelm, Beier, Dauster & Partner
(86) Internationale Anmeldenummer: PCT/EP2002/004492
(87) Internationale Veröffentlichungsnummer: WO 2002/088734

(56) Entgegenhaltungen:
- WO-A-02/27320
- WO-A-99/21958
- OLBRICH K ET AL: "Water permeability and mechanical strength of polyunsaturated lipid bilayers." BIOPHYSICAL JOURNAL, Bd. 79, Nr. 1, Juli 2000 (2000-07), Seiten 321-327, XP008008841 ISSN: 0006-3495 in der Anmeldung erwähnt

## Beschreibung

Die Erfindung betrifft poröse Partikel, die mit einer Lipidschicht umspannt sind, sowie die Verwendung dieser Partikel zur Messung der Membranpermeation von Substanzen.

In vielen Bereichen der Forschung ist es notwendig, die Membrangängigkeit verschiedener Substanzen zu charakterisieren. Das Verhalten in Bezug auf Membranen bzw. auf Lipide ist allgemein bei der Untersuchung von Biomolekülen, insbesondere von Peptiden oder Proteinen, ein wichtiger Aspekt. In ganz besonderem Maße spielt die Membrangängigkeit von Substanzen in der pharmazeutischen Forschung bei der Wirkstoffindung und Wirkstoffcharakterisierung eine große Rolle. Ein ganz entscheidender Punkt für die Einsetzbarkeit eines Wirkstoffes im Bereich der Medizin ist, inwieweit dieser Wirkstoff in der Lage ist, Membranen zu durchdringen und so beispielsweise in das Innere von Zellen zu gelangen. Für diesen Bereich, die sogenannte Pharmakokinetik, werden schon seit langem geeignete Modellsysteme gesucht und erforscht. Diese Modelle sollen es ermöglichen, die natürlichen Bedingungen im Organismus bezüglich der dort auftretenden Membranen soweit zu imitieren, daß anhand dieser Modellsysteme zuverlässige Aussagen über die Membrangängigkeit der jeweiligen Substanzen in vivo getroffen werden können.

Die Permeation von Substanzen durch Membranen bzw. durch Lipidschichten beruht im wesentlichen auf passiven und aktiven Transportmechanismen. Für den aktiven Transport sind verschiedene Elemente innerhalb der Membran bzw. der Lipidschicht von entscheidender Bedeutung. Hierbei handelt es sich vor allem um Transportproteine, die den Durchtritt verschiedener Substanzen durch eine Membran überhaupt erst ermöglichen. Wichtig in diesem Zusammenhang sind auch lonenkanäle in Membranen, die im wesentlichen auch von Proteinen gebildet sind, und welche den Durchtritt von Ionen ermöglichen und steuern.

Die bisher etablierten Methoden zu einer experimentellen Bestimmung der Permeation von Stoffen durch Membranen, insbesondere durch Biomembranen, können bezüglich der hierfür verwendeten Membranmorphologie in planare und sphärisch gekrümmte Membransysteme unterteilt werden.

Die planaren Membransysteme sind zumeist an der Kontaktstelle zwischen zwei ansonsten völlig getrennten, wässrigen Kompartimenten A und B angeordnet und erlauben die Messung des Durchgangs von Substanzen von Kompartiment A nach Kompartiment B durch herkömmliche Methoden. Zu diesen Membransystemen zählen die "black lipids membranes" (BLM) (Wardak, A., Brodowski, R., Krupa, Z., Gruszecki, W. I., (2000) Journal of Photochemistry and Photobiology B 56, 12-18), Membranen in Filterporen (Kansy, M., Sermer, F., Gubernator, K. (1998) Journal of Medicinal Chemistry 41, 1007-1010 und Schmidt, C., Mayer, M., Vogel, H., (2000) Angewandte Chemie Int. Edition 39, 3137-3140) sowie die Klasse der festkörperunterstützten Membranen (Cornell, B. A., Braach-Maksvyits, V. L., King, L. G., Osman, P. D., Raguse, B., Wiecorek, L., Pace, R. J., (1997) Nature 387, 580-583). Durch ihre Morphologie sind derartige Membransysteme für biosensorische Anwendungen einsetzbar. Allerdings ist die Größe des Kompartiments B bei den festkörperunterstützten Membranen im Vergleich zum Kompartiment A im allgemeinen sehr klein, wodurch eine Permeation von Stoffen durch die Membran in Folge der begrenzten Aufnahmekapazität von Kompartiment B beeinflußt werden kann.

Ein weiterer entscheidender Nachteil dieser planaren Membransysteme ist die insgesamt geringe Membranoberfläche, die für den Stoffaustausch zwischen den beiden Kompartimenten A und B zur Verfügung steht. Dies trifft in besonderem Maße auf die sogenannten Patch-Clamp-Techniken zu, bei denen Bereiche mikroskopischer Dimension natürlicher oder artifizieller Membranen über eine Pipettenspitze gespannt werden und der Stoff- bzw. Ionentransport elektrisch detektiert wird (Bordi, F., Cametti, C., Motta, A., (2000) Journal of Physical Chemistry B, 104, 5318-5323).

Aufgrund dieser Nachteile konnten planare Membransysteme bisher hauptsächlich nur zur Detektion der Permeation von Ionen durch Membranen sinnvoll genutzt werden. Lediglich planare Membranen in Filterporen konnten für die Messung der Permeation anderer Substanzen genutzt werden (Kansy, M., Sermer, F., Gubernator, K. (1998) Journal of Medicinal Chemistry 41,1007-1010). Ein generelles Problem dieser planaren Systeme ist jedoch immer die Undefiniertheit der jeweiligen Membranen bzw. Lipidschichten. Es ist hierbei nicht zu kontrollieren, ob es sich um beispielsweise eine Lipiddoppelschicht oder um sogenannte Multischichten handelt. Da derartige Permeationsmessungen durchgeführt werden, um Informationen über das Verhalten von Substanzen unter natürlichen Bedingungen zu erhalten, ist es unverzichtbar, daß mit definierten Lipidschichten, also insbesondere mit Lipiddoppelschichten, gearbeitet wird. Kann dies nicht gewährleistet werden, sind zum einen keine reproduzierbaren Ergebnisse zu erreichen, und zum anderen haben derartige Ergebnisse wenig Aussagekraft in Bezug auf Voraussagen über das Verhalten der Substanzen unter natürlichen Bedingungen.

Zu den sphärisch gekrümmten Membransystemen zählen die sogenannten Liposomen oder Vesikel, die ein äußeres Kompartiment A von einem inneren Kompartiment B abgrenzen. Dieses Kompartiment B befindet sich innerhalb der Liposomen bzw. Vesikel und befindet sich damit auch innerhalb des Kompartiments A. Diese Systeme haben aufgrund ihrer kolloiden Dimensionen gegenüber den planaren Systemen den Vorteil einer wesentlich größeren Membrangrenzfläche zwischen den Kompartimenten A und B, wodurch insbesondere die Messung langsam permeierender Stoffe ermöglicht wird. Außerdem kann hierdurch die Fehlerrate von Einzelmembranmessungen drastisch gesenkt werden. Permeationsmessungen mit diesen Systemen sind bisher unter Verwendung verschiedener Detektionsmethoden beschrieben worden. Hierzu zählt beispielsweise die Fluoreszenz (Sigler, A., Schubert, P., Hillen, W., Niederweis, M., (2000) European Journal of Biochemistry 267, 527-534), Radioaktivität sowie elektrische Meßmethoden (Hill, W. G., Zeidel, M. L., (2000) Journal of Biological Chemistry 275, 30176-30185). Diese Detektionsmethoden können für eine zeitaufgelöste Detektion der stattfindenden Permeation genutzt werden. Permeationsmessungen an einzelnen Liposomen sind ebenfalls beschrieben (Olbrich, K., Rawicz, W., Needham, D., Evans, E., (2000) Biophysical Journal 79, 321-327). Derartige Messungen sind jedoch technisch sehr aufwendig und fehleranfällig und daher nicht für routinemäßige Messungen geeignet.

Der generelle Nachteil der herkömmlichen sphärisch gekrümmten Membransysteme ist ihre Instabilität, welche zuverlässige und reproduzierbare Messungen, insbesondere im Rahmen von Reihenuntersuchungen, nahezu unmöglich macht. Weiterhin lassen sich die bekannten sphärisch gekrümmten Membransysteme bezüglich der Größe und Zahl der Lipidschichten morphologisch nicht definieren. Die Liposomen und Vesikel, die in diesem Zusammenhang genutzt werden, sind bezüglich der Morphologie vielmehr ein Zufallsprodukt, so daß zuverlässige, reproduzierbare Messungen im allgemeinen nicht möglich sind.

Um das Problem der Instabilität zu umgehen, wurde vorgeschlagen, für die Messung der Permeation mit Lipidmembranen beschichtete Hohlkugeln, die aus einem stabilen Netz hergestellt sind, zu verwenden (Moya, S., Donath, E., Sukhorukov, G. B., Auch, M., Baumler, H., Lichtenfeld, H., Möhwald, H., (2000) Macromolecules 33, 4538-4544). Hierdurch können relativ stabile Membransysteme bereitgestellt werden. Allerdings sind diese beschichteten Hohlkugeln nicht für eine automatisierte Messung der Membranpermeation von Stoffen geeignet. Zum einen ist es hier nicht möglich, das Kompartiment B, also das Innere der Hohlkugeln, mit verschiedenen Funktionalitäten auszustatten, die eine Detektion der permeierenden Stoffe erleichtern würden. Andererseits weisen die beschichteten Hohlkugeln eine so geringe Dichte auf, daß eine Isolierung der Kugeln aus beispielsweise einer wäßrigen Phase nur unter größerem Aufwand möglich ist. Eine solche Abtrennung des Membransystems wäre für eine schnelle und zuverlässige Analyse der permeierten Substanzen eine Voraussetzung.

In der internationalen Anmeldung WO 02/27320 ist beschrieben, durch geeignete Oberflächenoptimierungen eine Entkopplung der Membran von der Festkörperoberfläche zu erreichen, und damit "freien" Raum zwischen Membran und Festkörper einzustellen. Hierbei werden vergrößerte Oberflächen durch den Einsatz von porösen Partikeln zum Beschichten mit der Membran bereitgestellt, wobei die gesamte Oberfläche, also auch das Innere der Poren, mit Membran beschichtet wird. Der "freie" Raum zwischen Membran und Festkörper ist jedoch sehr klein und für Permeationsmessungen nicht geeignet.

Die Erfindung stellt sich daher die Aufgabe, ein Modellsystem für Membranen, insbesondere für native Membranen, bereitzustellen, mit welchem die Permeation von Substanzen durch Membranen bzw. Lipidschichten analysiert werden kann. Hierbei sollen die Membranen bzw. die Lipidschichten so genau definiert sein, daß zuverlässige und reproduzierbare Ergebnisse erzielt werden können. Weiterhin soll das System stabil sein. Das System soll darüber hinaus solche Eigenschaften aufweisen, daß es für automatisierte Prozesse geeignet ist. Schließlich stellt sich die Erfindung die Aufgabe, ein Membransystem zu schaffen, welches so flexibel ist, daß es den verschiedensten Versuchsbedingungen, insbesondere Detektionsverfahren, angepaßt werden kann.

Diese Aufgabe wird gelöst durch poröse Partikel, wie sie in Anspruch 1 beschrieben sind. Bevorzugte Ausführungsformen dieser Partikel sind in den Ansprüchen 2-15 ausgeführt. Die Ansprüche 16-27 betreffen ein Verfahren zur Herstellung der erfindungsgemäßen Partikel. Die Ansprüche 28-30 beschäftigen sich mit einem Verfahren zur Permeationsmessung von Substanzen unter Verwendung dieser Partikel. Die Ansprüche 31-33 betreffen die Verwendung der Partikel bzw. einen Kit zur Messung der Membranpermeation von Substanzen bzw. zur Untersuchung von Membrankomponenten. Der Wortlaut sämtlicher Ansprüche wird hiermit durch Bezugnahme zum Inhalt der Beschreibung gemacht.

Die erfindungsgemäßen porösen synthetischen Partikel weisen eine innerhalb ihrer Poren gebildete innere Oberfläche und eine äußere Oberfläche auf, die von der restlichen Oberfläche gebildet wird. Diese Partikel sind dadurch gekennzeichnet, daß sie von einer Lipidschicht vollständig bedeckt sind, wobei diese Lipidschicht die äußere Oberfläche der Partikel bedeckt und hierbei die Öffnungen der Poren an der äußeren Oberfläche überspannt. Die Poren besitzen erfindungsgemäß eine Öffnungsweite von etwa 3 bis etwa 50 nm. Die Lipidschicht dringt also im wesentlichen nicht in die Poren ein. Hierdurch wird ein System geschaffen, welches ein Kompartiment A außerhalb der Partikel von einem Kompartiment B innerhalb der Poren durch die Lipidschicht abtrennt. In Folge der Partikelstruktur des Systems handelt es sich hierbei um ein dispergierbares 2-Kompartiment-System. Dieses System ist in besonderer Weise für die Membranpermeationsmessung geeignet. Um die Membranpermeation der Stoffe zu untersuchen, wird das System mit Flüssigkeiten und den darin gelösten Stoffen in Kontakt gebracht. Die in den Flüssigkeiten gelösten Stoffe durchdringen in Abhängigkeit von ihren Membranpermeationseigenschaften die Lipidschicht und gelangen so in das Porenvolumen der Partikel. Nach dem Eintritt der Stoffe in das Porenvolumen können die permeierten Stoffe quantitativ analysiert werden, so daß auf diese Weise die Permeationskonstante der Stoffe bestimmt werden kann. Vorteilhafterweise umschließt die Lipidschicht die äußere Oberfläche der porösen Partikel im wesentlichen dicht. Dies ist notwendig, damit die zu analysierenden Substanzen nicht auf anderem Wege als über die Membran in die Poren eindringen können.

Bei der Lipidschicht, die die Partikel umspannt, handelt es sich vorzugsweise um eine Lipiddoppelschicht. Die Eigenschaften einer Lipiddoppelschicht kommen denen von nativen Membranen sehr nahe, so daß mit diesem erfindungsgemäßen System die natürlichen Bedingungen nachempfunden werden können. Im Gegensatz zu herkömmlichen Systemen kann im erfindungsgemäßen System die Morphologie der Lipidschicht sehr genau kontrolliert werden. Es handelt sich also um ein exakt definiertes System, welches die Voraussetzung für zuverlässige und reproduzierbare Versuchsergebnisse bildet. Von außen entspricht das erfindungsgemäße Partikelsystem in seiner Morphologie und seiner Oberflächenbeschaffenheit Liposomen bzw. Vesikeln, die herkömmlicherweise für Membranpermeationsmessungen verwendet werden. Neben anderen Vorteilen weist das erfindungsgemäße System allerdings eine wesentlich höhere Stabilität als Liposomen oder Vesikel auf und ist daher wesentlich besser für Membranpermeationsmessungen geeignet.

In einer besonders bevorzugten Ausführungsform der Erfindung ist zwischen der Oberfläche, insbesondere der äußeren Oberfläche, der Partikel, und der Lipidschicht eine Zwischenschicht vorgesehen. Bei dieser Zwischenschicht handelt es sich vorzugsweise um ein Netzwerk. Die Zwischenschicht bedeckt die Partikel, ohne im wesentlichen in die Poren einzudringen. Sie dient vornehmlich dazu, einen Träger für die Lipidschicht zu bilden, so daß die Lipidschicht im wesentlichen nur die äußere Oberfläche der Partikel bedeckt. Die Zwischenschicht ist so beschaffen, daß sie den diffusiven Transport von Lösungsmittel, insbesondere Wasser, und den darin gelösten Stoffen im Vergleich zu der Lipidschicht nicht wesentlich behindert. Zusätzlich ist die Zwischenschicht vorzugsweise relativ fest auf der Oberfläche der Partikel adsorbiert bzw. verankert, um eine entsprechende Langzeitstabilität der erfindungsgemäßen Partikel zu gewährleisten. Vorzugsweise ist die Zwischenschicht derart beschaffen, daß sie Wasser oder ein anderes Lösungsmittel aufnehmen kann. Die hierdurch einstellbare Dicke der Zwischenschicht stellt einen gewissen Abstand zwischen der Partikeloberfläche und der Lipidschicht her. Ein solcher Abstand ist im allgemeinen vorteilhaft, damit die dynamischen und strukturellen Eigenschaften der Lipidschicht nicht durch die Nähe zur Partikeloberfläche dominiert werden.

In einer bevorzugten Ausführungsform der Erfindung besteht die Zwischenschicht zumindest teilweise aus mindestens einem Polymer. Hierbei sind Polymere aus organischem Material besonders bevorzugt. Der Ausdruck Polymer umfaßt hier ebenfalls die Co-Polymere und die Block-Co-Polymere. Vorteilhafterweise handelt es sich bei den Polymeren um relativ langkettige Moleküle. Hierdurch wird gewährleistet, daß die Polymere schon aus sterischen Gründen die Öffnungen der Poren in der äußeren Oberfläche der Partikel überspannen und im wesentlichen nicht in die Poren eindringen. Als Polymere sind Polyelektrolyte, insbesondere anionische Polyelektrolyte, Polyampholyte, insbesondere Proteine, DNA und/oder RNA, und/oder Polyzwitterionen geeignet.

In einer weiteren bevorzugten Ausführungsform der Erfindung ist das Polymer Polystyrolsulfonat (PSS), insbesondere Natrium-Polystyrolsulfonat, und/oder Polystyrol-co-Maleinsäureanhydrid (PSPMA). Auch diese Materialien sind erfindungsgemäß sehr geeignet, da sie aufgrund ihrer langkettigen Struktur im wesentlichen nicht in die Poren eindringen und die äußere Oberfläche der Partikel mit einem Netzwerk umspannen. Weiterhin nehmen diese Polymere bis zu einem gewissen Maß Wasser und/oder andere Lösungsmittel auf und gewährleisten somit, daß ein gewisser Mindestabstand zwischen Partikeloberfläche und Lipidschicht vorhanden ist, so daß die dynamischen Eigenschaften der Lipidschicht nicht behindert werden.

Die Zwischenschicht kann aus einer Aufeinanderschichtung von verschiedenen Molekülen bestehen, wobei die Moleküle vorzugsweise miteinander wechselwirken. Bevorzugterweise ist die der Partikeloberfläche am nächsten liegende Schicht durch Adsorption und/oder Chemiesorption fixiert.

Die Dichte bzw. die Maschenweite der Zwischenschicht wird zum einen von dem gewählten Material für die Zwischenschicht beeinflußt. Andererseits hängt sie von den gewählten Herstellungsbedingungen der Zwischenschicht, insbesondere der Konzentration des Materials für die Zwischenschicht, ab. Die Dichte bzw. die Maschenweite der Zwischenschicht wird vorzugsweise so gewählt, daß die freie Diffusion der Stoffe nicht beeinträchtigt wird und daß die Trägerfunktion der Zwischenschicht gewährleistet ist. Somit kann es bevorzugt sein, daß die Maschenweite relativ groß ist. Andererseits kann es auch vorteilhaft sein, die Maschenweite enger zu wählen, so daß das gesamte System insgesamt stabiler ist. Hierdurch wird beispielsweise eine höhere Druckbeständigkeit erreicht. Dies kann im Hinblick auf das Arbeiten mit höheren osmotischen Gradienten und/oder im Zusammenhang mit Lager- und/oder Transporteigenschaften vorteilhaft sein.

In einer weiteren bevorzugten Ausführungsform weisen die Poren der Partikel Substanzen auf und/oder sind insbesondere mit den Substanzen im wesentlichen gefüllt. Hierfür geeignete Substanzen bewirken keine wesentliche Einschränkung des diffusiven Transportes von Stoffen innerhalb der Poren. Die Substanzen innerhalb der Poren erfüllen eine gewisse Stützfunktion für die Zwischenschicht und/oder die Lipidschicht. Das Material der Zwischenschicht, insbesondere die Polymere, müssen die Poren in diesem Fall nicht ohne Unterstützung überspannen. Es können daher auch relativ kurzkettige Materialien, insbesondere kurzkettige Polymere, für die Herstellung der Zwischenschicht geeignet sein. In einer besonders bevorzugten Ausführungsform der Erfindung kann aufgrund der Stützfunktion der Substanzen innerhalb der Poren auf die Zwischenschicht verzichtet werden, so daß die Lipidschicht direkt auf die äußere Oberfläche der Partikel immobilisiert ist, wobei auch hier die Porenöffnungen an der äußeren Oberfläche von der Lipidschicht überspannt sind. Die Ausführungsform mit Substanzen innerhalb der Poren hat den entscheidenden Vorteil, daß die Druckbeständigkeit des Systems deutlich erhöht sein kann.

Bevorzugterweise handelt es sich bei den Substanzen innerhalb der Poren um Polymere, insbesondere um Polymere aus organischem Material. Hierbei umfassen die Polymere auch Co-Polymere und Block-Co-Polymere. Besonders bevorzugt sind Polyelektrolyte, Polyampholyte, insbesondere Proteine, DNA und/oder RNA, und/oder Polyzwitterionen. Ganz besonders geeignet sind Fluoreszenzsonden und/oder Lumineszenzsonden, die für die Detektion der permeierten Stoffe bei Durchführung der Permeationsmessung genutzt werden können. In einer bevorzugten Ausführungsform der Erfindung sind die Substanzen innerhalb der Poren nicht wasserlöslich und können somit als Matrix für das Einbringen von weiteren hydrophoben Molekülen in die Poren dienen. Die Substanzen können durch Adsorption und/oder Chemisorption fixiert sein. Die Substanzen können weiterhin eine chemische Bindung, die Adsorption oder den Einschluß weiterer Moleküle ermöglichen. Bevorzugterweise weist die Zwischenschicht bzw, die Füllung der Poren weitere Moleküle, insbesondere funktionelle Moleküle, auf.

In einer bevorzugten Ausführungsform der Erfindung ist die Oberfläche, insbesondere die innere Oberfläche, der Partikel modifiziert. Hierdurch kann beispielsweise eine insgesamt hydrophobe (passive) Oberfläche bereitgestellt sein, die für das Aufbringen von weiteren Molekülen, insbesondere von Molekülen mit hydrophoben Funktionalitäten, durch Adsorption und/oder chemische Verbindung geeignet ist. Eine hydrophobe innere Oberfläche kann beispielsweise durch das Aufbringen einer Silanschicht erreicht werden. Neben einer solchen Passivierung kann beispielsweise auch eine Aktivierung bevorzugt sein, wodurch die Oberfläche derart vorbereitet wird, daß eine im wesentlichen gezielte chemische Reaktion mit anderen Molekülen, beispielsweise mit Proteinen, möglich ist. In einer solchen Ausführungsform kann die Oberfläche beispielsweise mit Bromcyan modifiziert sein. In weiteren bevorzugten Ausführungsformen ist die innere Oberfläche mit Amino-, Epoxy-, Halogenyl- und/oder Thiogruppen modifiziert. Für die Modifizierung werden beispielsweise Mercaptane und/oder Disulfide, insbesondere Alkyldisulfide, eingesetzt. Besonders bevorzugte Beispiele sind N-(2-Aminoethyl)-3-Aminopropyltrimethoxysilan (EDA), Polyethylenimin (PEI) und/oder Cysteamin, insbesondere Cysteaminhydrochlorid.

In einer bevorzugten Ausführungsform der Erfindung weist die Oberfläche, insbesondere die innere Oberfläche, funktionelle Moleküle auf. Diese funktionellen Moleküle können unmittelbar mit der Oberfläche der Partikel interagieren und so fixiert werden. Bevorzugterweise werden die funktionellen Moleküle jedoch über eine Wechselwirkung mit einer modifizierten Oberfläche fixiert. Dies hängt selbstverständlich unter anderem von dem jeweiligen Material bzw. der Oberfläche der Partikel und den aufzubringenden funktionellen Molekülen ab. Weiterhin können die funktionellen Moleküle aufgrund von Wechselwirkungen mit der Zwischenschicht bzw. mit der Füllung der Poren fixiert sein.

Durch die Verwendung von hydrophoben Funktionalitäten innerhalb der Partikel läßt sich erfindungsgemäß ein System realisieren, welches über ein hydrophiles Kompartiment A und über ein hydrophobes Kompartiment B verfügt, an dessen Grenzfläche sich eine Lipidschicht befindet, die den Transport-zwischen den unterschiedlichen Kompartimenten kontrolliert.

Bevorzugterweise handelt es sich bei den funktionellen Molekülen um Moleküle, die im Zusammenhang mit der Detektion der permeierten Substanzen im Zuge der Permeationsmessung stehen. Vorzugsweise sind die funktionellen Moleküle enzymatisch, optisch und/oder chemisch, insbesondere photochemisch, aktive Moleküle. Besonders bevorzugt sind hierbei Moleküle, die für eine Fluoreszenz- und/oder Lumineszenzdetektion der permeierten Stoffe geeignet sind. Ganz besonders bevorzugt ist hierbei die Detektionsmethode des resonanten Energietransfers, bei dem ein Fluoreszenzdonor- und ein Fluoreszenzakzeptormolekül miteinander in Wechselwirkung treten. Hierfür wird entweder das Donor- oder das Akzeptormolekül innerhalb der Partikel fixiert. Die zu analysierenden Stoffe stellen nun den entsprechenden Fluoreszenzpartner, also das Akzeptor- oder das Donormolekül dar. Nachdem die Stoffe in die Poren durch die Lipidschicht hindurch eingetreten sind, treten sie in Wechselwirkung mit dem jeweils anderen Partnermolekül und bewirken so ein Fluoreszenzsignal, welches analysiert werden kann.

Es ist keine Voraussetzung für die Erfindung, daß ausschließlich die innere Oberfläche der Partikel modifiziert und/oder funktionalisiert ist. Sollte auch die äußere Oberfläche der Partikel modifiziert bzw. funktionalisiert sein, so ist in jedem Fall gewährleistet, daß bei der Permeationsmessung die zu analysierenden Stoffe zunächst die Lipidschicht durchqueren müssen, bevor sie mit diesen Funktionalitäten in Wechselwirkung treten.

Die Lipidschicht, die die porösen Partikel umgibt, kann in sehr großem Ausmaß variiert sein. Es sind im Prinzip beliebige Zusammensetzungen der Schicht möglich, wobei die Schicht im wesentlichen aus amphiphilen Molekülen besteht.

Besonders bevorzugt ist eine Lipidschicht, die zumindest teilweise aus Lipiden, Lipidderivaten, lipidanalogen Substanzen und/oder aus nativen Membranen, insbesondere Plasmamembranen, besteht. Der Einsatz von nativen Membranen oder Bruchstücken derartiger Membranen als Bestandteil der Lipidschicht hat den Vorteil, daß hierdurch zum einen die natürliche Situation widergespiegelt wird. Zum anderen braucht diese natürliche Situation nicht im einzelnen analysiert zu sein, insbesondere im Hinblick auf die verschiedenen Bestandteile der Membranen.

In dieser Vielseitigkeit der Lipidschicht liegt ein entscheidender Vorteil der Erfindung, da durch die vorgegebene Partikelgeometrie sichergestellt ist, daß jegliche darauf aufgebrachte Lipidschichtzusammensetzung zu Kompartiment A die gleiche Form und Größe exponiert. Dies ist beispielsweise bei der aus dem Stand der Technik bekannten Verwendung von Liposomen oder Vesikeln nicht der Fall, da deren Morphologie von der Zusammensetzung der jeweiligen Lipidschicht entscheidend abhängt.

Es ist besonders bevorzugt, daß die Lipidschicht weitere Substanzen, insbesondere Peptide, Proteine, Nukleinsäuren, Tenside und/oder Polymere, aufweist. Durch derartige Zusammensetzungen der Lipidschicht können die natürlichen Bedingungen einer Membran nahezu identisch widergespiegelt werden. Somit stellt das erfindungsgemäße Membransystem ein optimales Modellsystem für natürliche Membranen bereit.

Weiterhin ist es erfindungsgemäß bevorzugt, daß die Lipidschicht Transportelemente aufweist. Hierzu zählen insbesondere Transportproteine, beispielsweise Peptidtransporter, Porenbildner und/oder Ionenkanäle. Unter Porenbildnern sind solche Substanzen zu verstehen, die Löcher in Membranen generieren. Bevorzugterweise kann die Lipidschicht in ihrer ganzen Dicke von speziellen Molekülen durchspannt sein, die eine Transportfunktion für in flüssigen Phasen gelöste Substanzen wahrnehmen können. Hierdurch ist es zum einen möglich, die natürlichen Bedingungen einer Membran zu imitieren. Andererseits ist es hierdurch möglich, die Wechselwirkung bestimmter Stoffe mit bestimmten Transportelementen gezielt zu analysieren. So kann beispielsweise untersucht werden, unter welchen Bedingungen ein Transportprotein oder ein Ionenkanal seine optimale Aktivität entfaltet.

In einer besonders bevorzugten Ausführungsform der Erfindung sind die porösen Partikel poröse Kugeln. Vorteilhafterweise haben die Kugeln einen Durchmesser von etwa 1 bis etwa 100 µm, insbesondere etwa 3 bis etwa 10 µm.

Geeignete nanoporöse Partikel besitzen vorzugsweise eine definierte Porengröße, so daß durch die Verwendung von Partikeln einer bestimmten Porengröße die Eigenschaften des erfindungsgemäßen Systems gezielt kontrolliert werden können. Vor allem in Abhängigkeit von den jeweils gewählten Detektionsmethoden kann es bevorzugt sein, größere oder kleinere Porengrößen bzw. andere Partikeldurchmesser zu verwenden. Beispielsweise kann durch einen relativ geringen Porendurchmesser eine erhöhte Sensitivität von eingebauten lokalen Sondenmolekülen, z. B. Farbstoffen, bewirkt werden. So sind für die bereits erwähnte Detektionsmethode des resonanten Energietransfers typische Wechsel-wirkungsabstände von Donor- und Akzeptormolekülen im Bereich von ca. 5 nm nahezu optimal. Bei einem Porendurchmesser der erfindungsgemäßen Partikel von beispielsweise 10 nm tritt damit jedes permeierte Molekül (z. B. Donormolekül) nach Durchtritt durch die Lipidschicht zwangsläufig mit den an der Porenwand immobilisierten Molekülen (z. B. Akzeptormolekülen) in Wechselwirkung. Durch eine Variation der Porendurchmesser ist auf diese Weise eine "Feineinstellung" der Wechselwirkung möglich.

In einer bevorzugten Ausführungsform der Erfindung wird die innere Oberfläche der Poren mit Fluoreszenzsonden bestückt, deren Fluoreszenz sensitiv auf die Nähe eines bestimmten Stoffes, insbesondere eines bestimmten Moleküls oder Ions, reagiert, welches von Kompartiment A nach Kompartiment B permeiert ist. Durch die große innere Oberfläche der porösen Partikel ist auf diese Weise eine hohe Fluoreszenzausbeute erreichbar, die zu einer empfindlichen, zeitaufgelösten Detektion des Permeationsprozesses mittels herkömmlicher fluoreszenzspektroskopischer Methoden ausgenutzt werden kann.

Darüber hinaus hat die Verwendung der erfindungsgemäßen porösen Partikel zur Membranpermeationsmessung den weiteren Vorteil, daß die Diffusion in den Poren aufgrund der geringen Größe der Poren im wesentlichen zweidimensional und somit schneller erfolgt als in den herkömmlichen dreidimensionalen Systemen. Durch geeignete Wahl der Porendurchmesser kann auch der mittlere Abstand der permeierten Stoffe zu den Sondenmolekülen eingestellt werden, was eine optimale Wechselwirkung und effiziente Detektion gewährleistet.

In einer Ausführungsform der Erfindung bestehen die porösen Partikel zumindest teilweise aus anorganischem Material, insbesondere aus Siliziumoxiden, Aluminiumoxiden und/oder Titanoxiden. In einer weiteren bevorzugten Ausführungsform bestehen die porösen Partikel zumindest teilweise aus organischem Material, vorzugsweise aus Latex.

In einer besonders bevorzugten Ausführungsform bestehen die porösen Partikel zumindest teilweise aus Silikat. Die an der Oberfläche von porösen Silikatpartikeln befindlichen SiOH-Gruppen können vorteilhafterweise zur Funktionalisierung der Oberfläche mit geeigneten Molekülen genutzt werden. Besonders bevorzugt sind hierbei kovalente Bindungen zwischen Oberflächengruppen und Molekülen. Außerdem können Moleküle mit positiver Überschußladung (z. B. Polykationen) durch Coulomb-Wechselwirkung fest an der Oberfläche adsorbiert werden. Die bei Silikatpartikeln extrem hohe Porosität der Partikel ermöglicht für das innere Kompartiment (Kompartiment B) eine im Vergleich zu den äußeren Abmessungen riesige innere Oberfläche, die für eine Fixierung von funktionellen Gruppen zur Verfügung steht. Poröses Silikat ist ein mechanisch festes Material mit einer negativen Oberflächenladung. Mikroskopische Partikel, insbesondere Kugeln, aus Silikat sind deshalb ausgezeichnet in Lösung, insbesondere in wäßriger Lösung, dispergierbar. Gleichzeitig sind die aufgrund ihrer Dichte zur Sedimentation unter normalen gravimetrischen Bedingungen fähig. Das bedeutet, daß bei Membranpermeationsmessungen bevorzugterweise auf eine Zentrifugation verzichtet werden kann. Nichtsdestotrotz kann unter bestimmten Bedingungen eine Zentrifugation vorteilhaft sein, um beispielsweise den Ablauf der Sedimentation zu verkürzen.

In einer weiteren bevorzugten Ausführungsform der Erfindung weisen die porösen Partikel einen magnetischen Kern auf. Besonders bevorzugt sind hierbei poröse Silikatkugeln mit einem magnetischen Kern. Hierdurch kann die Sedimentation der Partikel beschleunigt werden, beispielsweise im Hinblick auf eine Automatisierung des Verfahrens der Permeationsmessung.

Bei den erfindungsgemäß bevorzugten porösen Silikatpartikeln ist die Anordnung der Poren innerhalb der Partikel relativ zufällig. Es ist also nicht eindeutig definiert, ob die Poren vollständig miteinander kommunizieren oder nicht. Es kann jedoch vorteilhaft sein, Partikel einzusetzen, die derart definierte Poren aufweisen, daß gewährleistet ist, daß die Poren ein kommunizierendes System bilden. Vorteilhafterweise können sich hierbei Gleichgewichte innerhalb der Partikel schneller einstellen, und so bestimmte Reaktionen innerhalb der Partikel optimiert werden.

Die Erfindung umfaßt weiterhin ein Verfahren zu Herstellung von porösen synthetischen Partikeln mit einer innerhalb der Poren gebildeten inneren Oberfläche und einer übrigen äußeren Oberfläche, wobei die äußere Oberfläche von einer Lipidschicht, insbesondere von einer Lipiddoppelschicht, vollständig bedeckt ist, und die Lipidschicht die Öffnungen der Poren an der äußeren Oberfläche überspannt. Dieses Verfahren ist dadurch gekennzeichnet, daß die Poren der Partikel mit Substanzen versetzt, insbesondere im wesentlichen gefüllt, werden und/oder die porösen Partikel mit einer Schicht, insbesondere mit einem Netzwerk, versehen werden. In einem weiteren Verfahrensschritt werden die derart behandelten Partikel mit einer Lipidschicht, insbesondere mit einer Lipiddoppelschicht, versehen. Bezüglich verschiedener Einzelheiten des erfindungsgemäßen Verfahrens wird auf die obige Beschreibung verwiesen.

Vorteilhafterweise wird die Oberfläche, insbesondere die innere Oberfläche, der Partikel modifiziert, insbesondere passiviert oder aktiviert, wie es oben beschrieben ist. Weiterhin kann die Oberfläche mit funktionellen Molekülen, beispielsweise Sondenmolekülen, versehen werden (Funktionalisierung). Durch die Funktionalisierung können die durch die Modifizierung eingebrachten Gruppen "umfunktionalisiert" werden. Die Fixierung der funktionellen Moleküle kann beispielsweise durch Chemisorption oder Adsorption erfolgen. In einer bevorzugten Ausführungsform werden die Oberflächen zunächst modifiziert und/oder funktionalisiert und anschließend mit der Schicht, also der Zwischenschicht, versehen. Diese Zwischenschicht stellt vorzugsweise ein Netzwerk dar, welches insbesondere zumindest teilweise aus Polymeren besteht.

Erfindungsgemäß ist es besonders bevorzugt, die Modifizierung bzw. Funktionalisierung der Oberfläche nach einer erfolgten Beschichtung der Partikel vorzunehmen. Eine Voraussetzung für dieses Vorgehen ist, daß die Dichte bzw. die Maschenweite der Zwischenschicht ausreichend groß ist, um den Durchtritt der Modifizierungs- bzw. Funktionalisierungssubstanzen zu ermöglichen. Besonders bevorzugt ist diese Vorgehensweise bei der Verwendung von Sondenmolekülen, die für eine Fluoreszenzdetektion vorgesehen sind. Im allgemeinen hängt es von den jeweils gewählten Materialien, insbesondere dem Material der Zwischenschicht und dem Material für die Modifizierung bzw. Funktionalisierung, ab, ob diese Verfahrensabfolge vorteilhaft ist. Die nachträgliche Funktionalisierung bzw. Modifizierung der Oberfläche hat herstellungstechnisch sehr große Vorteile, da auf diese Weise das ganze Verfahren zur Herstellung der erfindungsgemäßen Partikel vereinfacht werden kann. Beispielsweise können alle Substanzen, die zur Herstellung der erfindungsgemäßen Partikel vor dem Aufbringen der Lipidschicht notwendig sind, in einem Ansatz auf die Partikel gegeben werden.

Nach dem Aufbringen der Schicht, also der Zwischenschicht und/oder nachdem die Poren der Partikel mit Substanzen versetzt bzw, im wesentlichen gefüllt wurden, wird eine Lipidschicht aufgebracht. Das Aufbringen der Lipidschicht erfolgt vorteilhafterweise nachdem eventuelle Modifizierungen und/oder Funktionalisierungen der Partikeloberflächen vorgenommen wurden. Für das Herstellen der Lipidschicht werden Vesikel aus Lipiden, Lipidderivaten, lipidanalogen Substanzen, nativen Membranen, insbesondere Plasmamembranen, hergestellt. Bei der Herstellung dieser Vesikel können auch weitere Substanzen, wie beispielsweise Peptide oder Proteine sowie Transportelemente anwesend sein, und damit in die Vesikel eingebaut werden. Die Herstellung der Vesikel und das Aufbringen der Lipidschicht auf die Partikel erfolgt nach herkömmlichen Methoden wie sie z. B. von Schmitt, J., Danner, B., Bayerl, T. M., (2000) Langmuir 17, 244-246 beschrieben sind.

Die Erfindung umfaßt ferner ein Verfahren zur Membranpermeationsmessung von Substanzen, wobei die erfindungsgemäßen porösen synthetischen Partikel verwendet werden. Hierfür werden die zu untersuchenden Substanzen mit den erfindungsgemäßen porösen Partikeln in einem Ansatz in Kontakt gebracht. Nach einer gewissen Inkubationszeit, welche vorteilhafterweise genau definiert ist, wird die Menge der Substanzen, die die Membran durchdrungen haben, analysiert. So können Aussagen zur Membranpermeationseigenschaft der jeweiligen Substanz getroffen werden.

Die Menge der Substanzen, die durch die Membran hindurch in das Innere der Partikel, insbesondere in die Poren, gelangt ist, wird direkt und/oder indirekt bestimmt. In einer besonders bevorzugten Ausführungsform dieses Verfahrens werden die Partikel nach der Inkubationszeit von dem übrigen Ansatz abgetrennt und die Menge der Substanzen, die sich innerhalb der Partikel befindet, bestimmt. In einer weiteren Ausführungsform erfolgt nach der Abtrennung der Partikel vom Ansatz eine Bestimmung der Menge der Substanzen im verbliebenen Ansatz. Beide Vorgehensweisen können vorteilhafterweise miteinander kombiniert werden. Die Abtrennung der Partikel kann auf verschiedene Weise erfolgen, beispielsweise durch Zentrifugation und/oder Filterung. Besonders bevorzugt ist eine "natürliche" Sedimention, da dies das Verfahren der Permeationsmessung wesentlich vereinfacht. Es kann jedoch vorteilhaft sein, das Verfahren zu beschleunigen, so daß eine Zentrifugation vorteilhaft sein kann.

In einer ganz besonders bevorzugten Ausführungsform der Erfindung werden magnetische Partikel wie oben beschrieben eingesetzt. Aufgrund dieser magnetischen Eigenschaft können die Partikel sehr schnell und effizient abgetrennt werden. Dies ist insbesondere im Hinblick auf eine Automatisierung des gesamten Verfahrens vorteilhaft. Bei Verwendung von magnetischen Partikeln werden die Partikel mit Hilfe eines geeigneten Magneten abgetrennt und der übrige Ansatz und die Partikel stehen getrennt voneinander für die weitere Analyse zur Verfügung. Hiermit entfällt der zeitraubende und einer Automatisierung schwer zugängliche Zentrifugationsschritt. Bei einer automatisierten Permeationsmessung kann das Verfahren beispielsweise in Mikrotiterplatten durchgeführt werden, wobei bereits eine Vielzahl von Automatisierungshilfsmitteln für solche Mikrotiterplatten zur Verfügung stehen. Vorzugsweise erfolgt dabei die Inkubation und die Detektion des Permeationsprozesses in demselben Gefäß.

In einer bevorzugten Ausführungsform des erfindungsgemäßen Verfahrens erfolgt die Bestimmung der zu analysierenden Substanzen durch chemische, elektrische, magnetische, radioaktive, optische, insbesondere fluorometrische oder luminometrische, Nachweis- bzw. Detektionsmethoden. Beispielsweise kann die Membranpermeation von fluoreszenzmarkierten Stoffen untersucht werden, indem die Stoffe in gelöster Form in das Kompartiment A, also zu den dispergierten Partikeln, gegeben werden. Nach definierten Inkubationszeiten werden die Partikel vom restlichen Ansatz abgetrennt und die Fluoreszenzintensität identischer Kugelmengen vorzugsweise als Funktion der Inkubationszeit mit herkömmlichen Meßmethoden bestimmt. In einer weiteren bevorzugten Ausführungsform des erfindungsgemäßen Verfahrens werden Fluoreszenzsonden verwendet, die im Kompartiment B fixiert sind. Deren Fluoreszenz ist sensitiv für bestimmte Stoffe, insbesondere Moleküle oder Ionen, die im Kompartiment A gelöst sind. Die Permeation dieser Moleküle oder Ionen vom Kompartiment A nach Kompartiment B bewirkt eine Veränderung der gesamten Fluoreszenzintensität der Dispersion. Dies kann als Funktion der Inkubationszeit gemessen werden. In dieser Ausführungsform ist es nicht notwendig, die Partikel vom übrigen Ansatz nach der Inkubationszeit abzutrennen.

In einer weiteren Ausführungsform werden radioaktiv markierte Stoffe im Kompartiment A eingesetzt. Nach erfolgter Inkubation wird die Detektion durch Bestimmung der von den Kugeln ausgehenden Strahlungsintensität mittels geeigneter radioaktiver Meßverfahren durchgeführt. Dieses Ausführungsbeispiel hat den Vorteil, daß die Detektionsmöglichkeiten extrem empfindlich sind, und daß der zu untersuchende Stoff lediglich gering durch den Einbau der jeweiligen Radioaktivität verändert wird.

In einer weiteren Ausführungsform der Erfindung werden Enzyme im Kompartiment B immobilisiert, so daß sie bevorzugterweise ihre Aktivität beibehalten. Durch permeierende Stoffe, die vom Kompartiment A in das Kompartiment B gelangen und damit in die Nähe der Enzyme, wird bewirkt, daß die Enzyme in einen anderen Zustand versetzt werden, der beispielsweise optisch, z. B. durch Fluoreszenz oder Lumineszenz, oder elektrisch detektierbar ist.

Das erfindungsgemäße Verfahren kann so durchgeführt werden, daß nach einer gewissen Inkubationszeit die permeierten Substanzen analysiert werden. Besonders bevorzugt ist es jedoch, wenn die Permeation als Funktion der Inkubationszeit bestimmt wird, d. h. also, daß die permeierten Substanzen entweder nach verschiedenen, definierten Inkubationszeiten als Endpunktbestimmung analysiert werden, oder daß die Permeation bei der Verwendung von geeigneten Nachweismethoden im laufenden Ansatz verfolgt wird.

In einer weiteren Ausführungsform werden Partikel verwendet, die im Kompartiment B Enzyme aufweisen, für welche die zu analysierenden Stoffe Edukte darstellen. Nach der Permeation der Stoffe setzt das Enzym diese in Produkte um. Die Freisetzung der Produkte im Kompartiment B führt entweder selbst zu einer optisch oder elektrisch detektierbaren Veränderung, oder das Enzym wird zu einer solchen Veränderung veranlaßt und/oder die Produkte sind durch andere Methoden nachweisbar.

In einer weiteren Ausführungsform werden nicht markierte Stoffe im Kompartiment A eingesetzt, deren Permeation untersucht werden soll. Hier kann eine Detektion durch Bestimmung der nach einer Inkubationszeit und Abtrennung der Partikel im Kompartiment A verbliebenen Menge des Stoffes mittels Methoden wie beispielsweise HPLC/UV-Vis-Spektroskopie oder HPLC/Massenspektroskopie. Ein entscheidender Vorteil dieses Ausführungsbeispiels ist, daß der zu untersuchende Stoff nicht markiert und/oder speziell gereinigt werden muß und dennoch mit hoher Empfindlichkeit nachgewiesen werden kann.

Weiterhin umfaßt die Erfindung die Verwendung von porösen synthetischen Partikel, wie sie oben beschrieben ist, zur Membranpermeationsmessung.

Weiterhin umfaßt die Erfindung einen Kit zur Messung der Membranpermeation von Substanzen. Dieser Kit enthält die notwendigen Komponenten, die zur Herstellung von erfindungsgemäßen porösen synthetischen Partikeln wie oben beschrieben geeignet sind.

Weiterhin umfaßt die Erfindung einen Kit zur Messung der Membranpermeation von Substanzen, wobei der Kit vorzugsweise vollständig beschichtete poröse synthetischen Partikel gemäß der obigen Beschreibung aufweist. Dies ist besonders bevorzugt, wenn die Partikel mit einer relativ komplizierten Lipidschicht versehen sind, also insbesondere einer Lipidschicht, die neben Lipiden noch weitere Substanzen aufweist wie beispielsweise Proteine, insbesondere Transportproteine.

Schließlich umfaßt die Erfindung einen Kit zur Untersuchung von Membranelementen, insbesondere von Proteinen. Mit dem erfindungsgemäßen Kit können vorteilhafterweise funktionelle Untersuchungen durchgeführt werden. Beispielsweise kann ein solcher Kit für eine Charakterisierung von Transportproteinen eingesetzt werden.

Die Erfindung hat gegenüber bisher bekannten Systemen bzw. Verfahren zur Membranpermeationsmessung entscheidende Vorteile. Dies gilt vor allem in Bezug auf Stabilität, mechanische Festigkeit, Morphologie, Detektion und Separierbarkeit aus einer Dispersion. Eine Automatisierung der Permeationsmessung ist in sehr vorteilhafter Weise möglich. Die erfindungsgemäßen Partikel zeichnen sich durch neuartige Möglichkeiten zur Funktionalisierung der Oberflächen und damit zu einsetzbaren Detektionsmöglichkeiten aus.

Die beschriebenen Merkmale der Erfindung sowie weitere Merkmale ergeben sich aus den nachfolgenden Beispielen, den Figuren und den Unteransprüchen. Hierbei können die verschiedenen Merkmale jeweils für sich oder in Kombination miteinander verwirklicht sein.

In den Figuren ist gezeigt:
- Fig. 1:: Differentialkalorimetrische-Meßkurven von mit Dielaidoylphosphatidyl-cholin (DEPC) beschichteten porösen Silikatpartikeln mit und ohne überspannten Poren,
- Fig. 2:: ATP-stimulierte Ca²⁺-Transportmessungen der immobilisierten Ca²⁺-ATPase des sarkoplasmatischen Retikulums bei lipidbeschichteten Silikatpartikeln mit und ohne Polymerzwischenschicht.

### Beispiele

### Beispiel 1: FUNKTIONALISIERUNG DER FESTKÖRPEROBERFLÄCHE

### 1.1. Aminofunktionalisierung von pulverförmigen und porösen Silikatoberflächen mit N-(2-Aminoethyl)-3-Aminopropyl-' trimethoxysilan (EDA)

Eine Silanlösung, bestehend aus 9,2 mL N-(2-Aminoethyl)-3-Aminopropyltrimethoxysilan (EDA) und 243 µL konzentrierter Essigsäure in 450 mL entionisiertem Wasser, wird frisch hergestellt. Nach 5 Minuten werden 2 g eines porösen Silikatmaterials (Nucleosil 50-10 von Macherey-Nagel, Düren) zu der Silanlösung gegeben und unter Schütteln aufgeschlämmt. Diese Dispersion wird drei Stunden langsam rotiert, danach wird das Silikatmaterial sedimentiert und dreimal mit entionisiertem Wasser gewaschen. Der Erfolg der Silanisierung wird mittels Infrarotspektroskopie in diffuser Reflexion (DRIFT) am getrockneten Silikatmaterial dokumentiert. In ähnlicher Weise werden weitere Trägermaterialien mit Porengrößen zwischen 5 und 400 nm funktionalisiert.

### 1.2. Aminofunktionalisierung von pulverförmigen und porösen Silikatoberflächen mit Poly(diallyl-dimethylammoniumchlorid)

Zu einer Poly(diallyl-dimethylammoniumchlorid) (PolyDADMAC, Mw etwa 400.000 - 500.000)-Lösung bestehend aus 200 mL Polyethylenimin (PEI) (20%-Lösung in Wasser, Aldrich, Steinheim) in 50 mL einer 3M NaCl-Lösung werden 1 g eines porösen Silikatmaterials (Nucleosil 50-3 von Macherey-Nagel, Düren) gegeben und drei Stunden langsam rotiert. Danach wird das Silikatmaterial sedimentiert und dreimal mit entionisiertem Wasser gewaschen. Der Erfolg der Umsetzung wird mittels Infrarotspektroskopie in diffuser Reflexion (DRIFT) am getrockneten Silikatmaterial dokumentiert.

### Beispiel 2: EINBAU FUNKTIONELLER MOLEKÜLE VOR DER ÜBERSPANNUNG

### 2.1. Immobilisierung eines Enzyms (Esterase)

1 g eines entsprechend Beispiel 1.1, funktionalisierten EDA-Trägermaterials werden in einer Esterase-Lösung, bestehend aus 30 mg ESTERASE (E.C. 3.1.1.1., Aktivität 20 units/mg) in 15 mL Phosphatpuffer (20 mM, pH 7.4) gegeben und über Nacht rotiert. Danach wird das Trägermaterial sedimentiert und dreimal mit Phosphatpuffer gewaschen. Der Erfolg der Behandlung wird durch die Abnahme der Esterase aus der Lösung und mittels Aktivitätsmessungen des immobilisierten Enzyms auf dem Trägermaterial dokumentiert.

### 2.2. Einbringen eines Sondenmoleküls

50 mg eines entsprechend Beispiel 1.1. funktionalisierten EDA-Trägermaterials werden in 1 mL einer 1 mM Quin2 (Calbiochem, Bad Soden)-Lösung in TEA-Puffer (50 mM TEA, 25 mM NaCl) gegeben und eine Stunde rotiert. Danach wird das Trägermaterial sedimentiert und dreimal mit TEA-Puffer gewaschen. Der Erfolg der Behandlung wird durch die Abnahme des Quin2 aus der Lösung und mittels Fluoreszenzmessungen dokumentiert.

### Beispiel 3: ÜBERSPANNEN DER PORÖSEN KUGELN MIT POLYMEREN

### 3.1. Adsorption von Na-Polystyrolsulfonat (PSS) auf EDA-funktionalisierten Siliktoberflächen

Zu einer Na-Polystyrolsulfonat (PSS)-Lösung, bestehend aus 25 mg PSS (Mw etwa 2.600.000, FLUKA) in 50 mL entionisiertem Wasser, wird 1 g eines entsprechend Beispiel 1.1. aminofunktionalisiertem Silikatmaterials gegeben und drei Stunden geschüttelt. Danach wird das Silikatmaterial sedimentiert und dreimal mit entionisiertem Wasser gewaschen. Der Erfolg der Adsorption wird mittels DRIFT und der Abnahme der PSS-Konzentration in der Lösung dokumentiert.

### 3.2. Adsorption von Na-Polystyrolsulfonat (PSS) auf Poly(diallyl-dimethyl-ammoniumchlorid)-funktionalisierten Silikatoberflächen

Zu einer Na-Polystyrolsulfonat (PSS)-Lösung, bestehend aus 25 mg PSS (Mw etwa 70.000, Aldrich, Steinheim) in 50 mL entionisiertem Wasser, wird 1 g eines entsprechend Beispiel 1.2. aminofunktionalisiertem Silikatmaterials gegeben und drei Stunden geschüttelt. Danach wird das Silikatmaterial sedimentiert und dreimal mit entionisiertem Wasser gewaschen. Der Erfolg der Adsorption wird mittels DRIFT und der Abnahme der PSS-Konzentration in der Lösung dokumentiert.

### 3.3. Adsorption von Na-Polystyrolsulfonat (PSS) auf enzymhaltigen Silikat-oberflächen

Zu einer Na-Polystyrolsulfonat (PSS)-Lösung bestehend aus 12,5 mg PSS (Mw etwa 2.600.000, FLUKA) in 25 mL entionisiertem Wasser, wird 0,5 g eines entsprechend Beispiel 2.1. hergestellten Silikatmaterial gegeben und drei Stunden geschüttelt. Danach wird das Silikatmaterial sedimentiert und dreimal mit entionisiertem Wasser gewaschen. Der Erfolg der Adsorption wird mittels DRIFT und der Abnahme der PSS-Konzentration in der Lösung dokumentiert. Die Integrität des Enzyms nach dem Überspannen wird durch Aktivitätsmessungen bestimmt.

### Beispiel 4: EINBAU FUNKTIONELLER MOLEKÜLE NACH DER ÜBERSPANNUNG

### 4.1. Herstellung photoreaktiver Oberflächen auf PSS/EDA-funktionalisierten Silikatoberflächen

0,25 g eines entsprechend Beispiel 3.1. funktionalisierten EDA/PSS-Trägermaterials wird in eine Lösung, bestehend aus 0,25 g 3,3',4,4'-Benzophenontetracarbonsäuredianhydrid (BPA) in 25 mL Aceton, gegeben und über Nacht rotiert. Danach wird das Trägermaterial sedimentiert, dreimal mit Aceton gewaschen und getrocknet. Der Erfolg der Behandlung wird mittels DRIFT dokumentiert.

### 4.2. Herstellung von Anhydridoberflächen auf PSS/EDA-funktionalisierten Silikat-oberflächen

0,25 g eines entsprechend Beispiel 3.1. funktionalisierten EDA/PSS-Trägermaterials wird in eine Lösung, bestehend aus 0,1 g 3,3'4,4'-Biphenyltetracarbonsäuredianhydrid in 25 mL Aceton, gegeben und über Nacht rotiert. Danach wird das Trägermaterial sedimentiert, dreimal mit Aceton gewaschen und getrocknet.

### 4.3. Einbringen eines Sondenmoleküls

50 mg eines entsprechend Beispiel 3.1. funktionalisierten EDA/PSS-Trägermaterials wird in 1 mL einer 1 mM Quin2 (Calbiochem, Bad Soden)-Lösung in TEA-Puffer (50 mM TEA,
25 mM NaCl, pH 7.4) gegeben und eine Stunde rotiert. Danach wird das Trägermaterial sedimentiert und dreimal mit TEA-Puffer gewaschen. Der Erfolg der Behandlung wird durch die Abnahme des Quin2 aus der Lösung und mittels Fluoreszenzmessungen dokumentiert.

### Beispiel 5: DEPOSITION VON LIPIDSCHICHTEN AUF DEN MODIFIZIERTEN OBERFLÄCHEN

### 5.1. Herstellung von Lipidvesikeln zur Immobilisierung auf pulverförmigen Oberflächen

80 mg Dielaidoylphosphatidylcholin (DEPC) wird in 16 mL Beschichtungspuffer, bestehend aus 20 mM HEPES-Puffer pH 7,1 mit 30 mM NaCl, eine halbe Stunde bei Raumtemperatur gequollen und anschließend 30 Minuten mit einem Stabbeschaller (Branson Sonorex) ultrabeschallt. Das Ergebnis ist eine klare Vesikeldispersion mit Vesikeldurchmessern im Bereich 20-80 nm. Die Bestimmung erfolgt mittels herkömmlicher dynamischer Laserlichtstreuung ("Particle-Sizing").

### 5.2. Immobilisierung von Lipidmembranen auf überspannten Silikatoberflächen

Zu 16 mL einer entsprechend Beispiel 5.1 hergestellten Vesikeldispersion wird 1 g eines entsprechend Beispiel 3.1. überspannten porösen Silikatträgers zugegeben und 30 Minuten langsam rotiert. Danach wird das Trägermaterial sedimentiert und dreimal mit Beschichtungspuffer gewaschen. Der Erfolg der Beschichtung wird mittels DSC an dem im Beschichtungspuffer dispergiertem Material (wie beschrieben bei C. Naumann, T. Brumm, T. M. Bayerl, Biophys. J., 1992, 63, 1314) und DRIFT (nach Trocknung des Materials) und durch Bestimmung der Lipidmenge dokumentiert.

### 5.3. Immobilisierung von Lipidmembranen auf enzymhaltigen Oberflächen mit gemischter Funktionalität

Zu 16 mL einer entsprechend Beispiel 5.1 hergestellten Vesikeldispersion wird 1 g eines entsprechend Beispiel 3.3. überspannten und enzymhaltigen porösen Silikatträgers gegeben und 30 Minuten langsam rotiert. Danach wird das Trägermaterial sedimentiert und dreimal mit Beschichtungspuffer gewaschen. Der Erfolg der Beschichtung wird mittels DSC an dem im Beschichtungspuffer dispergiertem Material und DRIFT (nach Trocknung des Materials) dokumentiert.

### 5.4. Immobilisierung von nativen Sarkoplasmatischen Retikulum (SR)-Membranen auf überspannten Silikatoberflächen und Messung der Ca²⁺-ATPase-Funktion

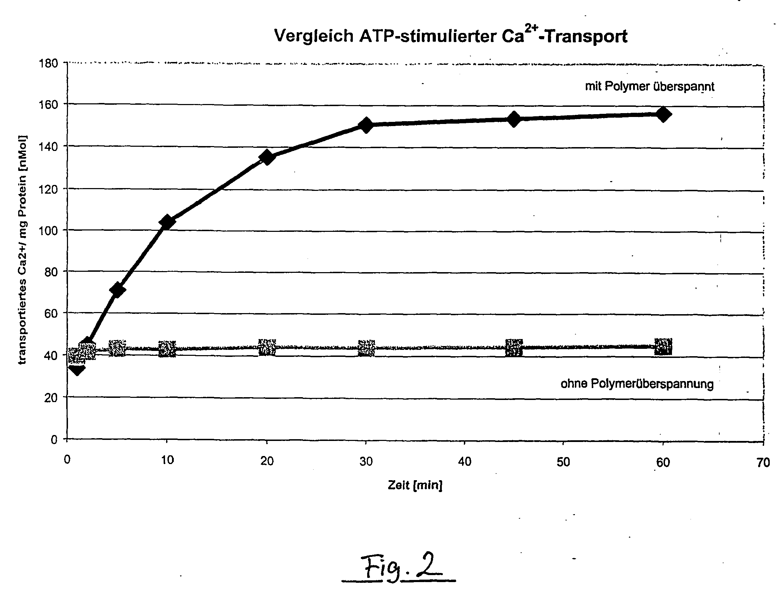

Aus dem Muskelgewebe eines Kaninchens werden nach einem Verfahren von W. Hasselbach und M. Makinose (Biochem. Z. 1961, 333, 518-528) Membranvesikel des sarkoplasmatischen Retikulums hergestellt (SR-Vesikel). Diese Dispersion wird anschließend durch Ultraschallbehandlung in kleine, einschalige Vesikel mit einem Durchmesser von 20-90 nm überführt. Zu 900 µL dieser Lösung (etwa 0,5 mg Gesamtprotein) werden 50 mg eines entsprechend Beispiel 4.3. überspannten porösen Silikatträgers zugegeben und für 18 Stunden bei 4°C inkubiert, wobei als Pufferlösung 100 mM Triethanolamin (pH 7,4) und 100 mM NaCl (Inkubationspuffer) verwendet wird. Danach wird das Trägermaterial sedimentiert und dreimal mit Inkubationspuffer gewaschen. Der Erfolg der Beschichtung wird mittels DRIFT am getrockneten Material dokumentiert. Die Ca²⁺-ATPase-Aktivität auf dem Trägermaterial nach der Waschung im Inkubationspuffer erfolgt durch Bestimmung der ATP-Hydrolyse-Aktivität in Abhängigkeit der Calciumionenkonzentration und des Ca²⁺⁻Transportes sowie dessen Hemmung durch den spezifischen Inhibitor "Cyclopiazonic Acid". Zur Messung der transportierten Menge an Ca²⁺-Ionen wurde die Fluoreszenz (Anregungsfilter: 340 nm, Emissionsfilter: 510 nm) in einem Fluoreszenzplattenleser (HTS7000, Perkin-Elmer) kontinuierlich gemessen (Figur 2). Diese Funktionstests belegen eine zum SR-Vesikel vergleichbare Ca²⁺-ATPase-Aktivität auf dem Trägermaterial.

### Beispiel 6: EIGENSCHAFTEN DER MEMBRANEN AUF OPTIMIERTEM TRÄGERMATERIAL

### 6.1. Stabilität im fließenden wäßrigen Medium

Die in Beispielen 5.2. bis 5.4. beschriebenen Systeme werden für die Dauer von 24 Stunden einem strömenden Medium (Beschichtungspuffer nach Beispiel 5.1. bzw. Inkubationspuffer nach Beispiel 5.4.) in einem Testbad ausgesetzt. In Abständen von 2 Stunden werden jeweils gleiche Mengen Trägermaterial aus dem Testbad entnommen, getrocknet und anschließend mittels DRIFT auf ihre Beschichtung untersucht. Die in den Beispielen 5.2. und 5.3. beschriebenen Systeme werden zusätzlich mittels DSC untersucht. Weder mit DRIFT noch mit DSC wird eine meßbare Abnahme der Membranbeschichtung mit der Zeit beobachtet.

### 6.2. Stabilität nach Einfrieren

Die in den Beispielen 5.2. bis 5.4. beschriebenen Systeme werden bei -80°C im dispergierten Zustand eingefroren und anschließend wieder auf Raumtemperatur gebracht und getrocknet. Vergleichende DRIFT-Messungen vor und nach dem Einfrieren ergaben unveränderte Lipidmengen auf dem Trägermaterial.

### 6.3. Stabilität der enzymatischen Aktivität von SR-beschichtetem Trägermaterial

Das in Beispiel 5.4. beschriebene System wird nach der Präparation über die Dauer von 3 Monaten bei -80°C aufbewahrt. Im Abstand von 1 Monat werden Proben entnommen und auf ihre Ca²⁺-ATPase-Aktivität mittels des in 5.4. beschriebenen Verfahrens untersucht. Nach 2 Monaten ist die Aktivität auf ca. 70 % des ursprünglichen Wertes (unmittelbar nach der Präparation und Waschung des Trägermaterials gemessen) abgesunken. Im Überstand der gelagerten Proben kann keine Ca²⁺-ATPase-Aktivität gemessen werden.

### 6.4. Bestimmung der Phasenübergangstemperaturen

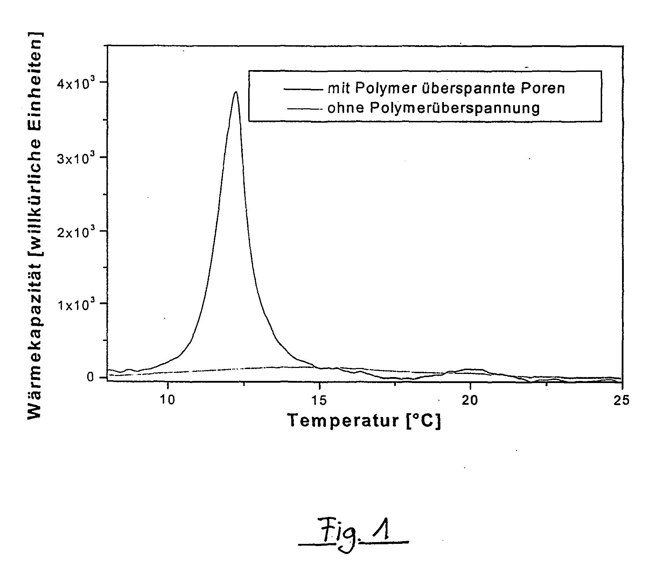

Figur 1 zeigt differentialkalorimetrische (DSC)-Messungen des Phasenübergangs festkörperunterstützter Bilayer, bestehend aus dem synthetischen Lipid Dielaidoyl-sn-3-glycero-3-phosphocholin (im folgenden DEPC genannt) auf einer nichtüberspannten Oberfläche (Herstellung gemäß C. Naumann, T. Brumm, T. M. Bayerl, Biophys. J., 1992, 63, 1314) und auf einer durch den obigen Schritt überspannten Oberfläche (wie im Beispiel beschrieben). Diese Ergebnisse zeigen eine deutliche Verbreiterung des Phasenübergangs bei der nichtüberspannten Oberfläche. Die Phasenübergangstemperatur auf der polymerüberspannten Oberfläche entspricht dem der DEPC-Vesikel.

## Patentansprüche

1. Poröse synthetische Partikel mit einer innerhalb der Poren gebildeten inneren Oberfläche und einer übrigen äußeren Oberfläche, wobei die äußere Oberfläche von einer Lipidschicht, insbesondere einer Lipiddoppelschicht, vollständig bedeckt ist, **dadurch gekennzeichnet, dass** die Lipidschicht die Öffnungen der Poren an der äußeren Oberfläche überspannt, wobei die Öffnungsweite der Poren von 3 bis 50 nm beträgt.

2. Partikel nach Anspruch 1, **dadurch gekennzeichnet, daß** zwischen der Oberfläche, insbesondere der äußeren Oberfläche, und der Lipidschicht eine Zwischenschicht, insbesondere ein Netzwerk, vorgesehen ist.

3. Partikel nach Anspruch 2, **dadurch gekennzeichnet, daß** die Zwischenschicht zumindest teilweise aus mindestens einem Polymer, insbesondere aus einem Polymer aus organischem Material, besteht.

4. Partikel nach Anspruch 3, **dadurch gekennzeichnet, daß** das Polymer ein Polyelektrolyt, insbesondere ein anionischer Polyelektrolyt, ein Polyampholyt, insbesondere ein Protein, DNA und/oder RNA, und/oder ein Polyzwitterion ist.

5. Partikel nach Anspruch 3 oder Anspruch 4, **dadurch gekennzeichnet, daß** das Polymer Polystyrolsulfonat (PSS), insbesondere Natrium-Polystyrolsulfonat, und/oder Polystyrol-co-Maleinsäureanhydrid (PSPMA) ist.

6. Partikel nach einem der vorhergehenden Ansprüche, **dadurch gekennzeichnet, daß** die Poren Substanzen aufweisen, insbesondere mit Substanzen im wesentlichen gefüllt sind, wobei vorzugsweise die Substanzen Polymere, insbesondere Polymere aus organischem Material, sind.

7. Partikel nach Anspruch 6, **dadurch gekennzeichnet, daß** die Polymere Polyelektrolyte, Polyampholyte, insbesondere Proteine, DNA und/oder RNA, Polyzwitterionen, Fluoreszenzsonden und/oder Lumineszenzsonden sind.

8. Partikel nach einem der vorhergehenden Ansprüche, **dadurch gekennzeichnet, daß** die Oberfläche, insbesondere die innere Oberfläche, Modifizierungen, insbesondere Passivierungen oder Aktivierungen, aufweist, wobei vorzugsweise die Modifizierungen Amino-, Epoxy-, Halogenyl- und/oder Thiogruppen sind.

9. Partikel nach einem der vorhergehenden Ansprüche, **dadurch gekennzeichnet, daß** die Oberfläche, insbesondere die innere Oberfläche, funktionelle Moleküle aufweist, wobei vorzugsweise die funktionellen Moleküle enzymatisch, optisch und/oder chemisch, insbesondere photochemisch, aktive Moleküle sind.

10. Partikel nach einem der vorhergehenden Ansprüche, **dadurch gekennzeichnet, daß** die Lipidschicht zumindest teilweise aus Lipiden, Lipidderivaten, lipidanalogen Substanzen und/oder nativen Membranen, insbesondere Plasmamembranen, besteht.

11. Partikel nach einem der vorhergehenden Ansprüche, **dadurch gekennzeichnet, daß** die Lipidschicht weitere Substanzen, insbesondere Peptide, Proteine, Nukleinsäuren, Tenside und/oder Polymere aufweist.

12. Partikel nach einem der vorhergehenden Ansprüche, **dadurch gekennzeichnet, daß** die Lipidschicht Transportelemente, insbesondere Transportproteine, Porenbildner und/oder Ionenkanäle, aufweist.

13. Partikel nach einem der vorhergehenden Ansprüche, **dadurch gekennzeichnet, daß** die Partikel poröse Kugeln sind, wobei vorzugsweise die Kugeln einen Durchmesser von 1 bis 100 µm, insbesondere 3 bis 10 µm, aufweisen.

14. Partikel nach einem der vorhergehenden Ansprüche, **dadurch gekennzeichnet, daß** die Partikel zumindest teilweise aus Silikat und/oder Latex bestehen.

15. Partikel nach einem der vorhergehenden Ansprüche, **dadurch gekennzeichnet, daß** die Partikel einen magnetischen Kern aufweisen.

16. Verfahren zur Herstellung von porösen synthetischen Partikeln mit einer innerhalb der Poren gebildeten inneren Oberfläche und einer übrigen äußeren Oberfläche, wobei die äußere Oberfläche von einer Lipidschicht, insbesondere einer Lipiddoppelschicht, vollständig bedeckt ist, und die Lipidschicht die Öffnungen der Poren an der äußeren Oberfläche überspannt, **dadurch gekennzeichnet, daß**
a) die Poren der Partikel eine Öffnungsweite von 3 bis 50 nm besitzen, und mit Substanzen versetzt, insbesondere im wesentlichen befüllt, werden und/oder die porösen Partikel mit einer Schicht, insbesondere mit einem Netzwerk, versehen werden und
b) daß auf die gemäß Verfahrensschritt a) behandelten Partikel eine Lipidschicht, insbesondere eine Lipiddoppelschicht, aufgebracht wird.

17. Verfahren nach Anspruch 16, **dadurch gekennzeichnet, daß** für das Versetzen der Poren mit Substanzen und/oder für das Herstellen der Schicht zumindest teilweise Polymere, insbesondere Polymere aus organischem Material, verwendet werden.

18. Verfahren nach Anspruch 17, **dadurch gekennzeichnet, daß** als Polymere Polyelektrolyte, insbesondere anionische Polyelektrolyte, Polyampholyte, insbesondere Proteine, DNA und/oder RNA, Polyzwitterionen, Fluoreszenzsonden und/oder Lumineszenzsonden eingesetzt werden.

19. Verfahren nach einem der Ansprüche 16 bis 18, **dadurch gekennzeichnet, daß** für das Herstellen der Schicht zumindest teilweise Polystyrolsulfonat (PSS), insbesondere Natrium-Polystyrolsulfonat, und/oder Polystyrol-co-Maleinsäureanhydrid (PSPMA) verwendet wird.

20. Verfahren nach einem der Ansprüche 16 bis 19, **dadurch gekennzeichnet, daß** die Oberfläche, insbesondere die innere Oberfläche, vor oder nach Durchführen des Verfahrensschrittes a) modifiziert, insbesondere passiviert oder aktiviert, wird, wobei vorzugsweise mit Amino-, Epoxy-, Halogenyl- und/oder Thiogruppen modifiziert wird.

21. Verfahren nach einem der Ansprüche 16 bis 20, **dadurch gekennzeichnet, daß** die Oberfläche, insbesondere die innere Oberfläche, vor oder nach Durchführen des Verfahrensschrittes a) mit funktionellen Molekülen versehen wird, wobei vorzugsweise als funktionelle Moleküle enzymatisch, optisch und/oder chemisch, insbesondere photochemisch, aktive Moleküle eingesetzt werden.

22. Verfahren nach einem der Ansprüche 16 bis 21, **dadurch gekennzeichnet, daß** für das Herstellen der Lipidschicht gemäß Verfahrensschritt b) Vesikel aus Lipiden, Lipidderivaten, lipidanalogen Substanzen, nativen Membranen, insbesondere Plasmamembranen, hergestellt werden und mit den Partikeln in Kontakt gebracht werden.

23. Verfahren nach einem der Ansprüche 16 bis 22, **dadurch gekennzeichnet, daß** für das Herstellen der Lipidschicht gemäß Verfahrensschritt b) die Vesikel weitere Substanzen, insbesondere Peptide, Proteine, Nukleinsäure, Tenside und/oder Polymere eingesetzt werden.

24. Verfahren nach einem der Ansprüche 16 bis 23, **dadurch gekennzeichnet, daß** für das Herstellen der Lipidschicht gemäß Verfahrensschritt b) weiterhin Transportelemente, insbesondere Transportproteine, Porenbildner und/oder Ionenkanäle, eingesetzt werden.

25. Verfahren nach einem der Ansprüche 16 bis 24, **dadurch gekennzeichnet, daß** als Partikel poröse Kugeln eingesetzt werden, wobei vorzugsweise die Kugeln einen Durchmesser von 1 bis 100 µm, insbesondere 3 bis 10 µm, aufweisen.

26. Verfahren nach einem der Ansprüche 16 bis 25, **dadurch gekennzeichnet, daß** Partikel eingesetzt werden, die zumindest teilweise aus Silikat und/oder Latex bestehen.

27. Verfahren nach einem der Ansprüche 16 bis 26, **dadurch gekennzeichnet, daß** Partikel eingesetzt werden, die einen magnetischen Kern aufweisen.

28. Verfahren zur Membranpermeationsmessung von Substanzen, **dadurch gekennzeichnet, daß**
a) die Substanzen mit porösen synthetischen Partikeln nach einem der Ansprüche 1 bis 15 in einem Ansatz in Kontakt gebracht werden und
b) nach einer Inkubationszeit die Menge der Substanzen innerhalb der Partikel direkt und/oder indirekt bestimmt wird.

29. Verfahren nach Anspruch 28, **dadurch gekennzeichnet, daß** nach der Inkubationszeit die Partikel von dem Ansatz abgetrennt werden, und die Menge der Substanzen innerhalb der Partikel und/oder im verbliebenen Ansatz bestimmt wird.

30. Verfahren nach Anspruch 28 oder Anspruch 29, **dadurch gekennzeichnet, daß** die Bestimmung der Substanzen durch chemische, radioaktive, optische, insbesondere fluorometrische oder luminometrische, Nachweismethoden erfolgt.

31. Verwendung von porösen synthetischen Partikeln nach einem der Ansprüche 1 bis 15 zur Membranpermeationsmessung.

32. Kit zur Messung der Membranpermeation von Substanzen, enthaltend die notwendigen Komponenten zur Herstellung der porösen synthetischen Partikel gemäß der Ansprüche 1-15.

33. Kit zur Untersuchung von Membranelementen insbesondere von Proteinen, enthaltend die notwendigen Komponenten zur Herstellung der porösen synthetischen Partikel gemäß der Ansprüche 1-15.

## Claims

1. Porous synthetic particles having an internal surface, which is formed within the pores, and a remaining external surface, with the external surface being completely covered by a lipid layer, in particular a lipid double layer, **characterized in that** the lipid layer spans the openings of the pores at the external surface, the opening width of the pores being from 3 to 50 nm.

2. Particles according to Claim 1, **characterized in that** an intermediate layer, in particular a network, is provided between the surface, in particular the external surface, and the lipid layer.

3. Particles according to Claim 2, **characterized in that** the intermediate layer consists at least partially of at least one polymer, in particular of a polymer composed of organic material.

4. Particles according to Claim 3, **characterized in that** the polymer is a polyelectrolyte, in particular an anionic polyelectrolyte, a polyampholyte, in particular a protein, DNA and/or RNA, and/or a polyzwitterion.

5. Particles according to Claim 3 or Claim 4, **characterized in that** the polymer is poly(styrenesulfonate) (PSS), in particular sodium poly(styrenesulfonate), and/or poly(styrene)-co-maleic anhydride (PSPMA).

6. Particles according to one of the preceding claims, **characterized in that** the pores contain compounds, and are in particular essentially filled with compounds, with the compounds preferably being polymers, in particular polymers composed of organic material.

7. Particles according to Claim 6, **characterized in that** the polymers are polyelectrolytes, polyampholytes, in particular proteins, DNA and/or RNA, polyzwitterions, fluorescence probes and/or luminescence probes.

8. Particles according to one of the preceding claims, **characterized in that** the surface, in particular the internal surface, exhibits modifications, in particular passivations or activations, with the modifications preferably being amino, epoxy, halogenyl and/or thio groups.

9. Particles according to one of the preceding claims, **characterized in that** the surface, in particular the internal surface, exhibits functional molecules, with the functional molecules preferably being enzymically, optically and/or chemically, in particular photochemically, active molecules.

10. Particles according to one of the preceding claims, **characterized in that** the lipid layer consists at least partially of lipids, lipid derivatives, lipid-analogous substances and/or native membranes, in particular plasma membranes.

11. Particles according to one of the preceding claims, **characterized in that** the lipid layer contains other substances, in particular peptides, proteins, nucleic acids, surfactants and/or polymers.

12. Particles according to one of the preceding claims, **characterized in that** the lipid layer contains transport elements, in particular transport proteins, pore formers and/or ion channels.

13. Particles according to one of the preceding claims, **characterized in that** the particles are porous spheres, with the spheres preferably having a diameter of from 1 to 100 µm, in particular from 3 to 10 µm.

14. Particles according to one of the preceding claims, **characterized in that** the particles consist at least partially of silicate and/or latex.

15. Particles according to one of the preceding claims, **characterized in that** the particles possess a magnetic core.

16. Process for producing porous synthetic particles having an internal surface, which is formed within the pores, and a remaining external surface, with the external surface being completely covered by a lipid layer, in particular a lipid double layer, and the lipid layer spanning the openings of the pores at the external surface, **characterized in that**
a) the pores of the particles have an opening width of from 3 to 50 nm and are spiked, in particular essentially filled, with compounds and/or the porous particles are provided with a layer, in particular with a network, and
b) a lipid layer, in particular a lipid double layer, is applied to the particles which have been treated in accordance with process step a).

17. Process according to Claim 16, **characterized in that** polymers, in particular polymers composed of organic material, are at least partially used for spiking the pores with compounds and/or for preparing the layer.

18. Process according to Claim 17, **characterized in that** the polymers employed are polyelectrolytes, in particular anionic polyelectrolytes, polyampholytes, in particular proteins, DNA and/or RNA, polyzwitterions, fluorescence probes and/or luminescence probes.

19. Process according to one of Claims 16 to 18, **characterized in that** poly(styrenesulfonate) (PSS), in particular sodium poly(styrenesulfonate), and/or poly(styrene)-co-maleic anhydride (PSPMA) is/are at least partially used for preparing the layer.

20. Process according to one of Claims 16 to 19, **characterized in that** the surface, in particular the internal surface, is modified, in particular passivated or activated, before or after implementing process step a), with amino, epoxy, halogenyl and/or thio groups preferably being used for the modification.

21. Process according to one of Claims 16 to 20, **characterized in that** the surface, in particular the internal surface, is provided with functional molecules before or after implementing process step a), with the functional molecules employed preferably being enzymically, optically and/or chemically, in particular photochemically, active molecules.

22. Process according to one of Claims 16 to 21, **characterized in that**, for preparing the lipid layer in accordance with process step b), vesicles are prepared from lipids, lipid derivatives, lipid-analogous substances, native membranes, in particular plasma membranes, and brought into contact with the particles.

23. Process according to one of Claims 16 to 22, **characterized in that** further substances, in particular peptides, proteins, nucleic acid, surfactants and/or polymers, are employed in preparing the vesicles for preparing the lipid layer in accordance with process step b).

24. Process according to one of Claims 16 to 23, **characterized in that** transport elements, in particular transport proteins, pore formers and/or ion channels, are also employed for preparing the lipid layer in accordance with process step b).

25. Process according to one of Claims 16 to 24, **characterized in that** the particles employed are porous spheres, with the spheres preferably having a diameter of from 1 to 100 µm, in particular of from 3 to 10 µm.

26. Process according to one of Claims 16 to 25, **characterized in that** use is made of particles which consist at least partially of silicate and/or latex.

27. Process according to one of Claims 16 to 26, **characterized in that** use is made of particles which possess a magnetic core.

28. Process for measuring the membrane permeation of substances,
**characterized in that**
a) the substances are brought into contact, in one mixture, with porous synthetic particles according to one of Claims 1 to 15, and
b) after an incubation period, the quantity of the substances present within the particles is determined directly and/or indirectly.

29. Process according to Claim 28, **characterized in that**, after the incubation period, the particles are separated off from the mixture and the quantity of the substances within the particles and/or in the remaining mixture is determined.

30. Process according to Claim 28 or Claim 29, **characterized in that** the substances are determined using chemical, radioactive or optical, in particular fluorimetric or luminometric, detection methods.

31. Use of porous synthetic particles according to one of Claims 1 to 15 for measuring membrane permeation.

32. Kit for measuring the membrane permeation of substances, comprising the necessary components for producing the porous synthetic particles according to Claims 1-15.

33. Kit for investigating membrane elements, in particular proteins, comprising the necessary components for producing the porous synthetic particles according to Claims 1-15.

## Revendications

1. Particules synthétiques poreuses ayant une surface interne formée à l'intérieur des pores et une surface externe restante, la surface externe étant recouverte complètement d'une couche lipidique, en particulier d'une double couche lipidique, **caractérisées en ce que** la couche lipidique recouvre les ouvertures des pores sur la surface externe, la largeur d'ouverture des pores étant de 3 à 50 nm.

2. Particules selon la revendication 1, **caractérisées en ce qu'**une couche intermédiaire, en particulier un réseau, est prévue entre la surface, en particulier la surface externe, et la couche lipidique.

3. Particules selon la revendication 2, **caractérisées en ce que** la couche intermédiaire est constituée au moins partiellement d'au moins un polymère, en particulier d'un polymère de matière organique.

4. Particules selon la revendication 3, **caractérisées en ce que** le polymère est un polyélectrolyte, en particulier un polyélectrolyte anionique, un polyampholyte, en particulier une protéine, un ADN et/ou un ARN, et/ou un polyzwitterion.

5. Particules selon la revendication 3 ou la revendication 4, **caractérisées en ce que** le polymère est le poly(sulfonate de styrène) (PSS), en particulier le poly(sulfonate de styrène) sodique, et/ou le copolymère styrène/anhydride maléique (PSPMA).

6. Particules selon l'une quelconque des revendications précédentes, **caractérisées en ce que** les pores présentent des substances, en particulier ils sont remplis essentiellement de substances, les substances étant de préférence des polymères, en particulier des polymères de matière organique.

7. Particules selon la revendication 6, **caractérisées en ce que** les polymères sont des polyélectrolytes, des polyampholytes, en particulier des protéines, des ADN et/ou des ARN, des polyzwitterions, des sondes à fluorescence et/ou des sondes à luminescence.

8. Particules selon l'une quelconque des revendications précédentes, **caractérisées en ce que** la surface, en particulier la surface interne, présente des modifications, en particulier des passivations ou des activations, les modifications étant de préférence des groupes amino, époxy, halogényle et/ou thio.

9. Particules selon l'une quelconque des revendications précédentes, **caractérisées en ce que** la surface, en particulier la surface interne, présente des molécules fonctionnelles, les molécules fonctionnelles étant de préférence des molécules à action enzymatique, optique et/ou chimique, en particulier, photochimique.

10. Particules selon l'une quelconque des revendications précédentes, **caractérisées en ce que** la couche lipidique est constituée au moins en partie de lipides, de dérivés lipidiques, de substances analogues aux lipides et/ou de membranes natives, en particulier de membranes plasmatiques.

11. Particules selon l'une quelconque des revendications précédentes, **caractérisées en ce que** la couche lipidique présente d'autres substances, en particulier des peptides, des protéines, des acides nucléiques, des tensio-actifs, et/ou des polymères.

12. Particules selon l'une quelconque des revendications précédentes, **caractérisées en ce que** la couche lipidique présente des éléments de transport, en particulier des protéines de transport, des agents porogènes et/ou des canaux ioniques.

13. Particules selon l'une quelconque des revendications précédentes, **caractérisées en ce que** les particules sont des sphères poreuses, les sphères présentant de préférence un diamètre de 1 à 100 µm, en particulier de 3 à 10 µm.

14. Particules selon l'une quelconque des revendications précédentes, **caractérisées en ce que** les particules sont constituées au moins en partie de silicates, et/ou de latex.

15. Particules selon l'une quelconque des revendications précédentes, **caractérisées en ce que** les particules présentent un noyau magnétique.

16. Procédé de préparation de particules synthétiques poreuses ayant une surface interne formée à l'intérieur des pores et une surface externe restante, la surface externe étant complètement recouverte d'une couche lipidique, en particulier d'une double couche lipidique, et la couche lipidique recouvrant les ouvertures des pores sur la surface externe, **caractérisé en ce que**,
a) les pores des particules possèdent une largeur d'ouverture de 3 à 50 nm, et sont bouchées avec des substances, en particulier remplies essentiellement de celles-ci, et/ou les particules poreuses sont pourvues d'une couche, en particulier d'un réseau et
b) une couche lipidique, en particulier une double couche lipidique, est appliquée sur les particules traitées selon l'étape de procédé a).

17. Procédé selon la revendication 16, **caractérisé en ce qu'**au moins en partie des polymères, en particulier des polymères de matière organique, sont employés pour boucher les pores avec des substances, et/ou pour la fabrication de la couche.

18. Procédé selon la revendication 17, **caractérisé en ce que** des polyélectrolytes, en particulier des polyélectrolytes anioniques, des polyampholytes, en particulier des protéines, des ADN et/ou des ARN, des polyzwitterions, des sondes à fluorescence et/ou des sondes à luminescence sont mis en oeuvre en tant que polymères.

19. Procédé selon l'une quelconque des revendications 16 à 18, **caractérisé en ce qu'**au moins en partie un poly(sulfonate de styrène) (PSS), en particulier un poly(sulfonate de styrène) sodique, et/ou un copolymère styrène/anhydride maléique (PSPMA) sont employés pour la fabrication de la couche.

20. Procédé selon l'une quelconque des revendications 16 à 19, **caractérisé en ce que** la surface, en particulier la surface interne, est modifiée, en particulier passivée ou activée, de préférence avec des groupes amino, époxy, halogényle et/ou thio, avant ou après réalisation de l'étape de procédé a).

21. Procédé selon l'une quelconque des revendications 16 à 20, **caractérisé en ce que** la surface, en particulier la surface interne, est pourvue de molécules fonctionnelles, de préférence des molécules à action enzymatique, optique et/ou chimique, en particulier photochimique, étant mises en oeuvre de préférence en tant que molécules fonctionnelles, avant ou après réalisation de l'étape de procédé a).

22. Procédé selon l'une quelconque des revendications 16 à 21, **caractérisé en ce que** des vésicules lipidiques, des dérivés lipidiques, des substances analogues à des lipides, des membranes natives, en particulier des membranes plasmatiques, sont préparées et amenées en contact avec les particules pour fabriquer la couche lipidique selon l'étape de procédé b).

23. Procédé selon l'une quelconque des revendications 16 à 22, **caractérisé en ce que** d'autres substances, en particulier des peptides, des protéines, un acide nucléique, des tensio-actifs et/ou des polymères, sont mises en oeuvre pour préparer les vésicules pour préparer la couche lipidique selon l'étape de procédé b).

24. Procédé selon l'une quelconque des revendications 16 à 23, **caractérisé en ce que** des éléments de transport, en particulier des protéines de transport, des agents porogènes, et/ou des canaux ioniques, sont mis en oeuvre en sus pour la préparation de la couche lipidique selon l'étape de procédé b).

25. Procédé selon l'une quelconque des revendications 16 à 24, **caractérisé en ce que** des sphères poreuses sont mises en oeuvre en tant que particules, les sphères présentant de préférence un diamètre de 1 à 100 µm, en particulier de 3 à 10 µm.

26. Procédé selon l'une quelconque des revendications 16 à 25, **caractérisé en ce que** des particules qui sont constituées au moins en partie de silicate et/ou de latex, sont mises en oeuvre.

27. Procédé selon l'une quelconque des revendications 16 à 26, **caractérisé en ce que** des particules qui présentent un noyau magnétique, sont mises en oeuvre.

28. Procédé pour mesurer la perméation membranaire de substances, **caractérisé en ce que**
a) les substances ayant des particules synthétiques poreuses selon l'une quelconque des revendications 1 à 15 sont amenées en contact dans un mélange, et
b) après une durée d'incubation, la quantité des substances à l'intérieur des particules est déterminée directement et/ou indirectement.

29. Procédé selon la revendication 28, **caractérisé en ce que**, après la durée d'incubation, les particules sont séparées du mélange et la quantité des substances à l'intérieur des particules et/ou dans le mélange restant, est déterminée.

30. Procédé selon la revendication 28 ou la revendication 29, **caractérisé en ce que** la détermination des substances se réalise au moyen de procédés de détection chimiques, radioactifs, optiques, en particulier fluorimétriques, ou luminométriques.

31. Utilisation de particules synthétiques poreuses selon l'une quelconque des revendications 1 à 15 pour la mesure de perméation membranaire.

32. Nécessaire pour la mesure de la perméation membranaire de substances, contenant les composants nécessaires à la préparation des particules synthétiques poreuses selon les revendications 1 à 15.

33. Nécessaire pour étudier des éléments membranaires, en particulier de protéines, contenant les composants nécessaires à la préparation des particules synthétiques poreuses selon les revendications 1 à 15.
